# EUROPEAN PATENT APPLICATION

(11) **EP 3 916 149 A1**
(43) Date of publication of application: **01.12.2021**
(21) Application number: 20176329.9
(22) Date of filing: 25.05.2020
(51) Int. Cl.: D21C 1/04, D21C 3/04, D21C 3/22, D21C 7/12, D21B 1/36, D21C 11/00

(54) **METHOD AND ARRANGEMENT FOR CONTROLLED PRETREATMENT OF BIOMASS**

(71) Applicant: Sekab E-Technology AB, 891 26 Örnsköldsvik (SE)
(72) Inventor: SJÖBLOM, Anders, 891 78 BONÄSSUND (SE); SUNDVALL, Elias, 892 32 DOMSJÖ (SE); HÄGGLUND, Karin, 892 92 DOMSJÖ (SE); CAVKA, Adnan, 891 96 ARNÄSVALL (SE)
(74) Representative: Kransell & Wennborg KB

(57) **Abstract**

The present disclosure generally relates to a method for controlled pretreatment of lignocellulosic biomass. The method comprises the steps of: Pretreating (S10) a lignocellulosic biomass material in a pretreatment arrangement, the pretreating comprising impregnating (S10A) the lignocellulosic biomass with an SO2 feed in an impregnation vessel of the pretreatment arrangement; collecting (S20) a number of process parameters of the pretreatment, which process parameters include at least a feed parameter related to the total amount of lignocellulosic biomass input to the pretreatment arrangement, and a dry matter parameter related to the dry matter content of lignocellulosic biomass input to the pretreatment arrangement; and adjusting (S30) the SO2 feed in response to the process parameters.

## Description

### TECHNICAL FIELD

The present disclosure generally relates to a method for controlled pretreatment of lignocellulosic biomass, and to a pretreatment arrangement for pretreatment of lignocellulosic biomass material.

### BACKGROUND

Lignocellulosic residues from forestry are attractive as feedstocks for the production of green chemicals and fuels, since they are abundant, relatively inexpensive, and not used for food. Lignocellulose consists mainly of lignin and two classes of polysaccharides, cellulose and hemicellulose. The polysaccharides can be hydrolyzed to sugars and converted to various fermentation products, e.g. bioalcohols, by means of fermenting microorganisms, such as Saccharomyces cerevisiae.

The hydrolysis of cellulose is typically preceded by a pretreatment process, in which the hemicellulose is degraded, and the cellulose is made more accessible to cellulolytic enzymes. During hydrolysis, the cellulose present is partly converted into reducing sugars.

The pretreatment is considered a critical part in the process of converting biomass into fermentation products, mainly because it affects downstream processes and determines the ultimate sugar yields. A variety of pretreatment processes exist, many of which rely on high temperature treatments and high pressures.

The pretreatment process is typically carried out in a pretreatment arrangement, comprising an impregnation vessel and a reactor vessel. In the impregnation vessel, the biomass is impregnated with an additive facilitating degradation of the biomass, and in the reactor vessel the degradation is improved by exposing the biomass to an elevated temperature and pressure. The pretreatment arrangement also generally comprises an inlet for receiving the biomass to be pretreated and an outlet for discharging the pretreated biomass, typically as a biomass slurry.

The overall aim of the pretreatment is to disrupt the crystalline cellulose structure and to remove or partially remove lignin from the lignocellulosic biomass. The pretreatment process is complex and involves many reactions and side-reactions. Such reactions may result in the formation of various by-products, which may be inhibitory to downstream processes. Furthermore, volatile compounds and gases contained in the lignocellulose biomass are set free when the material is degraded or partially degraded.

Pretreatment under harsh conditions is associated with problems such as undesirable fluctuations in conditions within the pretreatment arrangement. Such fluctuations may e.g. be associated with additives added during pretreatment. The stability of the process conditions within the pretreatment arrangement is thereby impaired. An effect of such imbalanced or unstable reaction conditions is that the pretreatment process becomes less efficient, i.e. provides a lower yield. Another effect is that deposits may form on the reactor walls.

There is a need for improvements with respect to a pretreatment system that is stable and controlled and which allows for the pretreatment process to be carried out in a consistent and reliable manner.

### SUMMARY

In view of the above, it is an object of the present invention to provide improvements with respect to methods and arrangements for pretreatment of lignocellulosic biomass, particularly to achieve a more stable process, for at least some process parameters, during operation.

According to a first aspect of the present invention, a method for controlled pretreatment of lignocellulosic biomass is provided. The method comprises the steps of:
- pretreating a lignocellulosic biomass material in a pretreatment arrangement, the pretreating comprising impregnating the lignocellulosic biomass with an SO2 feed in an impregnation vessel of the pretreatment arrangement;
- collecting a number of process parameters of the pretreatment, which process parameters include at least a feed parameter related to the total amount of lignocellulosic biomass input to the pretreatment arrangement, and a dry matter parameter related to the dry matter content of lignocellulosic biomass input to the pretreatment arrangement; and
- adjusting the SO2 feed in response to the process parameters.

Hereby, the impregnation of the lignocellulosic biomass by SO2 (sulfur dioxide) can be adapted based on at least the dry matter content, and total amount, of lignocellulosic biomass, in order to achieve a controllable and stable pretreatment process. Stated differently, by using at least the dry matter and feed parameter as process parameter regulating the SO2 feed, the pretreatment of the lignocellulosic biomass, and particularly the impregnation of the lignocellulosic biomass by SO2, can be controlled in an advantageous manner. In other words, problems with unstable pretreatment conditions related to unsatisfactory impregnation can be omitted, or at least reduced.

Without being bound by any theory, the dry matter content of the lignocellulosic biomass is believed to be at least notably correlated to the amount of SO2, or SO2 feed, and the resulting level of impregnation. This may, again without being bound by any theory, be at least partly related to moisture barriers within the lignocellulosic biomass.

The present invention provides a controlled SO2 feed to the impregnation vessel, preferably through an adjustable valve, and based on known data of the amount of lignocellulosic biomass fed to the pretreatment arrangement, or impregnation vessel, as well as the dry matter content of such lignocellulosic biomass, resulting in a controlled impregnation process. The feed parameter and dry matter parameter have been identified as key process parameters, together sufficient for controlling the SO2 feed and thereby achieve stable impregnation conditions.

According to at least one example embodiment, the collecting of the feed parameter and/or dry matter parameter is/are achieved by measurements. For example, the feed parameter may be determined by measuring the weight (kg) of the lignocellulosic biomass input to the pretreatment arrangement, or impregnation vessel. For a continuous feed of lignocellulosic biomass to the pretreatment arrangement, this may easily be converted into a mass flow (kg/h). The feed parameter may alternatively be determined by measuring the volume of the lignocellulosic biomass input to the pretreatment arrangement, or impregnation vessel. Correspondingly, the dry matter parameter may be determined by measuring the dry matter, or moisture, of the lignocellulosic biomass input to the pretreatment arrangement, or impregnation vessel. This may e.g. be achieved by an optical measurement, such as e.g. using IR or X-ray means. Alternatively, the feed parameter and/or dry matter parameter may be given by a lignocellulosic biomass provider, based on known data of the provided lignocellulosic biomass.

According to at least one example embodiment, the step of collecting a number of process parameters comprises measuring and monitoring the process parameters in a continuous or semi-continuous manner, and wherein the step of adjusting the SO2 feed comprises automatically adjusting a valve opening regulating the flow of SO2, in response to the process parameters.

According to at least one example embodiment, the step of adjusting the SO2 feed in response to the process parameters is adapted to meet a desired pH downstream of the impregnation vessel.

Hereby, the pH of the biomass material downstream of the impregnation vessel can be held at a desired value, or within a desired range, by adjusting the SO2 feed accordingly (based on the feed parameter and dry matter parameter). In other words, by knowing the amount, and dry matter content, of lignocellulosic biomass input to the pretreatment arrangement or impregnation vessel, and knowing the desired pH which the impregnation of the SO2 aims to achieve, the SO2 feed can be adapted accordingly. By controlling the pretreatment process, and particularly the operation of the impregnation vessel, to achieve a desired pH downstream of the impregnation vessel, the pretreatment process can be carried out in a stable and controllable manner. Thus, desired pH may be included as a process parameter.

It should be understood that downstream of the impregnation vessel is typically referring to a position between the impregnation vessel and the end of (or discharge from) the pretreatment arrangement, e.g. prior to any further downstream process steps as e.g. hydrolyzing. The exact position of the desired pH is not important, but the position typically reflects the resulting pH of the biomass caused by impregnation. For example, the desired pH may be at a position between the outlet of the impregnation vessel, and a downstream process step causing a change in pH.

According to at least one example embodiment, the step of pretreating a lignocellulosic biomass material in a pretreatment arrangement comprises subjecting SO2 impregnated biomass from the impregnating step to an elevated temperature and pressure in a reactor vessel of the pretreatment arrangement to form a pretreated biomass slurry.

Hereby, the lignocellulosic biomass undergoes degradation or partial degradation, in a known manner. By adapting the SO2 feed as previously described, the pretreatment process in the reactor vessel can be kept stable, and the degradation of the biomass optimized.

According to at least one example embodiment, the desired pH downstream of the impregnation vessel, may e.g. be at a position prior, at/in or after the reactor vessel.

According to at least one example embodiment, the method further comprises the steps of measuring pH of SO2 impregnated biomass from the impregnating step and/or the pretreated biomass slurry, determining whether or not the measured pH is within a predetermined range of the desired pH, and adjusting the SO2 feed if the measured pH is outside of the predetermined range of the desired pH.

That is, adjusting the SO2 feed in response to determining that the measured pH is outside of the predetermined range of the desired pH. Hereby, an effective means for adapting the SO2 feed in response to the actual pH (i.e. relating to the effectiveness of the impregnation) and desired pH is provided. Thus, measured pH may be included in the process parameters.

The pH may be measured on the biomass material (e.g. SO2 impregnated biomass out from the impregnation vessel, and/or the biomass slurry in the reactor vessel and/or the pretreated biomass slurry discharged from the reactor vessel), or on water/liquid separated from the biomass and removed from the process, e.g. liquid filtrate or hydrolysate.

According to at least one example embodiment, the desired pH is in the range of 1 to 4, preferably 1.2 to 2, and or more preferably 1.3 to 1.8.

Such pH is suitable for the pretreatment of the lignocellulosic biomass. According to at least one example embodiment, the step of impregnating the lignocellulosic biomass with an SO2 feed comprises supplying the SO2 to the impregnation vessel in gaseous form.

By utilizing the SO2 in gaseous form, the SO2 can be transported into the biomass material in a more efficient manner, compared to utilizing the SO2 in liquid form. Thus, impregnation of the lignocellulosic biomass is improved.

However, according to at least one example embodiment, the SO2 feed is alternatively, or additionality, provided as a liquid.

According to at least one example embodiment, the method further comprises the step of determining a relation between of the feed parameter and the dry matter parameter, and adjusting the SO2 feed in response to the determined relation.

Such relation of the feed parameter and the dry matter parameter provides a reliable and effective process parameter, which the inventors have realized is a good parameter to use when adapting the SO2 feed, especially when a desired pH is to be reached. The relation represents the inter-relation of the feed parameter and the dry matter parameter, or the combination of the feed parameter and the dry matter parameter. The relation may e.g. be a product parameter, for example a product between the feed parameter and dry matter parameter, potentially scaled with a suitable constant. The product thus relates to the total amount of dry matter biomass. Depending on the feed parameter and dry matter parameter, the relation may be a ratio. The relation is advantageously included into a model. According to at least one example embodiment, the determined relation is a determined correlation between the feed parameter and the dry matter parameter, and SO2 feed is adjusted in response to the determined correlation.

According to at least one example embodiment, the step of adjusting the SO2 feed comprises determining whether or not the determined relation is within a predetermined reference relation for the pretreatment; and adjusting the SO2 feed in response to determining that the determined relation is outside of the predetermined reference relation, such as by more than 5%, e.g. by more than 10% or more than 15 %.

Hereby, an effective way of adjusting the SO2 feed is provided.

According to at least one example embodiment, the method further comprises the steps of adjusting the moisture in the lignocellulosic biomass material prior to impregnating the lignocellulosic biomass with an SO2 feed in the impregnation vessel, and measuring the dry matter content of the lignocellulosic biomass prior to, and/or subsequent to, the adjusting moisture step, and wherein at least one of the measured dry matter contents is used as dry matter parameter.

Thus, the dry matter parameter may be based on the dry matter content of the lignocellulosic biomass fed to the impregnation vessel. Hereby, an improved correlation to the SO2 feed can be achieved.

According to at least one example embodiment, the adjusting moisture step comprises dewatering of the lignocellulosic biomass, e.g. carried out by compressing the lignocellulosic biomass. Compressing may e.g. be carried out by a plug screw feeder. According to at least one example embodiment, the adjusting moisture step comprises diluting the lignocellulosic biomass, e.g. by adding water.

According to at least one example embodiment, the step of pretreating comprises operating the impregnation vessel at a temperature of between 20 °C and 60 °C.

Thus, when adjusting the SO2 feed as previously described, the operating temperature in the impregnation vessel can be kept at a moderate level, while still reaching the desired pretreatment of the lignocellulosic biomass. According to at least one example embodiment, the step of pretreating comprises operating the impregnation vessel at a temperature of between 20 °C and 40 °C. However, according to at least one alternative embodiment, the step of pretreating comprises operating the impregnation vessel at a temperature of between 20 °C and 90 °C.

According to at least one example embodiment, the method further comprises the steps of retrieving at least a portion of SO2 from the pretreatment arrangement, and re-using the SO2 in the controlled pretreatment of the lignocellulosic biomass, or using the SO2 in a downstream process of the pretreatment arrangement.

Hereby, a more efficient use of SO2 is provided. The SO2 may e.g. be retrieved from the reactor vessel and/or the impregnation vessel, and re-used to impregnate the lignocellulosic biomass in the impregnation vessel.

According to at least one example embodiment, the method further comprises a steam explosion step, preferably arranged downstream, or in an end section of, the pretreatment arrangement.

For example, the desired pH downstream of the impregnation vessel may be relatively low, e.g. between 1 to 2, in embodiments utilizing a steam explosion step.

According to at least one example embodiment, the process parameters further include an SO2 parameter indicating the amount of SO2, or concentration of SO2 gas, in the impregnation vessel, or flow of SO2 entering the impregnation vessel.

Hereby, secondary SO2, in addition to the previously mentioned SO2 feed, which may be used in the impregnation of the lignocellulosic biomass can be taken into account when adjusting the SO2 feed.

According to at least a second aspect of the invention, a pretreatment arrangement for pretreatment of lignocellulosic biomass material is provided. The pretreatment arrangement comprises:
- an impregnation vessel having an upstream inlet for receiving a lignocellulosic biomass feed, a downstream outlet for discharging SO2-impregnated biomass, and an SO2 injection arrangement configured to supply an SO2 feed to the lignocellulosic biomass in the impregnation vessel, the SO2 injection arrangement comprising a valve having an adjustable opening configuration for varying the SO2 feed;
- a reactor vessel having an upstream inlet for receiving SO2-impregnated biomass from the impregnation vessel, and a downstream outlet for discharging the biomass as a pretreated biomass slurry;
- a control unit configured to collect a number of process parameters of the pretreatment arrangement, which process parameters include at least a feed parameter related to the amount of lignocellulosic biomass fed input to the pretreatment arrangement, and a dry matter parameter related to the dry matter content of lignocellulosic biomass fed input to the pretreatment arrangement, wherein the control unit is further configured to adjust the valve of the SO2 injection arrangement, and thereby adjust the SO2 feed, in response to the process parameters.

Effects and features of this second aspect of the invention are largely analogous to those described above in connection with the first aspect of the invention. Embodiments mentioned in relation to the first aspect of the invention are largely compatible with the second aspect of the invention, of which some are exemplified below (typically without repeating the advantageous effects).

According to at least one example embodiment, the pretreatment arrangement further comprises a pH measuring unit arranged and configured to measure the pH downstream of the impregnation vessel, and wherein the control unit is configured to adjust the SO2 feed in response to the process parameters, and to meet a desired pH downstream of the impregnation vessel.

The pH measuring unit may for example be arranged between the outlet of the impregnation vessel and inlet of the reactor vessel, in the reactor vessel, and/or downstream of the outlet of the reactor vessel. The pH measuring unit may be configured to measure the pH of the biomass or biomass slurry, or of any liquid withdrawn from the biomass and removed from the process.

According to at least one example embodiment, the pretreatment arrangement further comprises a moisture adjusting unit, e.g. compressing unit such as a plug screw feeder, configured to adjust the moisture content of the lignocellulosic biomass material prior to the inlet of the impregnation vessel, and comprising a measuring means arranged and configured to determine the dry matter content of the lignocellulosic biomass prior to, and/or subsequent to, the moisture adjusting unit, and wherein at least one of the measured dry matter contents is used as dry matter parameter.

Alternatively, the moisture adjusting unit is a configured to dilute the lignocellulosic biomass material by adding e.g. water.

According to at least one example embodiment, the pretreatment arrangement further comprises a steam explosion configuration arranged downstream, or in an outlet region of, the reactor vessel.

According to at least one example embodiment, the control unit is configured to perform at least one of the method steps of the first aspect of the invention.

According to at least a third aspect of the invention, a system for treatment of lignocellulosic biomass is provided. The system comprise a pretreatment arrangement according to the second aspect of the invention, a hydrolysis unit arranged downstream of and in fluid communication with the pretreatment arrangement, and optionally, a fermentation unit, such as a fermentation vessel, arranged downstream of and in fluid communication with the hydrolysis unit.

Effects and features of this third aspect of the invention are largely analogous to those described above in connection with the second aspect of the invention. Embodiments mentioned in relation to the second aspect of the invention are largely compatible with at least the pretreatment arrangement of the third aspect of the invention.

For example, the system may comprise additional units and components known to those skilled in the art. For example, a separation unit may be arranged between the pretreatment arrangement and the hydrolysis unit, and/or between the hydrolysis unit and the fermentation unit.

According to at least a fourth aspect of the invention, related to the retrieving of SO2, a method for preparing an SO2 based liquid derivable from an acidified lignocellulosic biomass is provided. The method of such fourth aspect comprises the steps of:
pretreating a lignocellulosic biomass material in a pretreatment arrangement, the pretreating including treating the lignocellulosic biomass material with SO2 and subjecting SO2 impregnated biomass to an elevated temperature and pressure in a reactor vessel of the pretreatment arrangement to provide a pretreated biomass slurry;
optionally hydrolyzing the pretreated biomass slurry in a hydrolysis unit with at least one aqueous hydrolyzing liquid, comprising saccharification enzymes, under conditions in which at least a part of the pretreated biomass slurry is hydrolyzed to a hydrolysate, said hydrolysate comprising fermentable sugars and inhibitory substances;
retrieving gaseous SO2 from the pretreatment arrangement to provide an SO2 gas stream;
treating the SO2 gas stream with an alkaline solution to provide a sulfite/bisulfite solution;
supplying at least a part of the sulfite/bisulfite solution to at least one of:
   a) the lignocellulosic biomass material prior to pretreating the lignocellulosic biomass material in the pretreatment arrangement;
   b) the lignocellulosic biomass material in the pretreatment arrangement, e.g. the reactor vessel;
   c) the pretreated biomass slurry discharged from the reactor vessel;
   d) the hydrolysate or fermentable sugars of the hydrolysate.

Hereby, an efficient yet simple way of re-using at least some substances from the process are provided. Besides the advantageous effects presented below for each one of the options a) - d), by re-using at least some substances a cost-effective process is provided. Moreover, by re-using at least some substances from the process, required cleaning and removal of substances e.g. due to emission restrictions, can be reduced or even omitted. In particular, the invention provides in addition to the process advantages also an efficient way of reducing SO2 (sulfur dioxide) related emissions. The pretreatment arrangement in this fourth aspect of the invention is advantageously the pretreatment arrangement discussed in relation to the first, second or third aspect of the invention.

The sulfite/bisulfite solution may thus be used in the process according to any one of (e.g. according to several of) options a) - d). Stated differently, the method comprises the step of supplying at least a part of the sulfite/bisulfite solution to the process upstream of, to, and/or downstream of the pretreatment of the lignocellulosic biomass material. At least a part of the sulfite/bisulfite solution may e.g. be at least 50 %, or at least 75 %, such as e.g. approximately 100 % or 100 % of the sulfite/bisulfite solution. According to at least one example embodiment, at least a part of the sulfite/bisulfite solution is less than 50 %, or less than 25 %, such as e.g. approximately 10 % of the sulfite/bisulfite solution.

According to at least one example embodiment, the step of pretreating the lignocellulosic biomass material in a pretreatment arrangement comprises treating the lignocellulosic biomass material with SO2 in an impregnation vessel of the pretreatment arrangement, and subjecting SO2 impregnated biomass from the impregnating step to the elevated temperature and pressure in the reactor vessel. According to at least one example embodiment, the step of pretreating the lignocellulosic biomass material in a pretreatment arrangement comprises providing SO2 pre-impregnated lignocellulosic biomass and subjecting the SO2 pre-impregnated lignocellulosic biomass to the elevated temperature and pressure in the reactor vessel.

In accordance with option a) the sulfite/bisulfite solution is used at least to impregnate the lignocellulosic biomass material in order to facilitate the mechanism in which the hemicellulose is degraded, and the cellulose is made more accessible to cellulolytic enzymes. Moreover, by impregnating the lignocellulosic biomass prior to the pretreatment arrangement, less SO2 may be needed in the impregnation step in the pretreatment, or the impregnation step in the pretreatment may be omitted. Thus, according to at least one example embodiment, the impregnation step using SO2 in the pretreatment arrangement may be omitted, and the sulfite/bisulfite treated lignocellulosic biomass material may be supplied directly to the reactor vessel of the pretreatment arrangement.

In accordance with option b) the sulfite/bisulfite solution may be used to impregnate the lignocellulosic biomass material for the same reasons mentioned in option a), but by supplying the sulfite/bisulfite solution to the lignocellulosic biomass material in the pretreatment arrangement, e.g. into the impregnation vessel. Here, the sulfite/bisulfite solution may be used as a complement to the SO2 supplied to the impregnation vessel. Additionality or alternatively, the sulfite/bisulfite solution can be supplied to the reactor vessel as makeup liquid (for dilution). Hereby, SO2 can be re-used in the process, as well as reducing the need of other makeup liquids (as e.g. makeup water). Moreover, this may lead to a reduced need of handling residue liquids, such as residue water, downstream of the pretreatment arrangement.

In accordance with options a) and/or b), the sulfite/bisulfite solution is typically subject to an environment with a low pH and/or high temperature, either as a direct effect of the point of application/supply (i.e. at a) or b)) or as a separate means arranged prior to the point of application/supply, which results in the formation of SO2 in gaseous form, facilitating impregnation of the lignocellulosic biomass material.

In accordance with option c) the sulfite/bisulfite solution is used at least to modify the characteristics of the pretreated biomass slurry prior to subjecting it to downstream process steps. Such modification is desired as the pretreated biomass slurry typically is not optimized for the downstream process steps, which is further discussed below. The supply of the sulfite/bisulfite solution according to option c) may be performed by supplying the sulfite/bisulfite solution to a process location downstream the reactor vessel, e.g. between the reactor vessel and the hydrolyzing unit.

In accordance with option d) the sulfite/bisulfite solution may be used at least to prevent undesired microbial growth of the fermentable sugars to facilitate storing of the same. For example, the fermentable sugars may initially be brought to a higher concertation than the target concentration, and subsequently be subject to dilution by the sulfite/bisulfite solution to reach the target concentration while additionally providing for the previously mentioned prevention of undesired microbial growth.

According to at least one example embodiment, the method comprises the step of detoxifying the hydrolysate by decreasing the concentration of at least one of the inhibitory substances using the sulfite/bisulfite solution.

Hereby, the resulting hydrolysate is less toxic with regards to yeast and/or other enzymes used in downstream process steps. It should be noted that the inhibitory substances are typically still present in the hydrolysate after the dilution with the sulfite/bisulfite solution, but that the inhibitory substances undergo reactions resulting in less toxic states.

According to at least one example embodiment, the step of detoxifying the hydrolysate is carried out by option c) above. However, it should be noted that any upstream supply of the sulfite/bisulfite solution at least to some extent contributes to the advantageous effects mentioned in relation to any downstream option of supply of the sulfite/bisulfite. For example, even if the sulfite/bisulfite solution is supplied in accordance with option a) or b) the advantageous effects of option c) or the detoxification of the hydrolysate, are still to at least some degree met, as the substances of the sulfite/bisulfite solution are following the flow of biomass further downstream of the point of application.

According to at least one example embodiment, the method comprises the step of increasing the pH of the pretreated biomass slurry to between 4 and 6, using the sulfite/bisulfite solution.

Hereby, a desired pH, e.g. with regards to preferred pH for enzymes, for the downstream process steps is provided. Moreover, the use of another means, such as e.g. an alkaline solution, for increasing the pH can be reduced or even omitted. However, it should be understood that the sulfite/bisulfite solution may be used together with such means (e.g. another alkaline solution) to reach the desired pH. For example, the sulfite/bisulfite solution may have a pH between 4 and 7, such as e.g. between 5 and 6, while the pretreated biomass slurry may have a pH of between 1 and 2, such as e.g. between 1.3 and 1.8. By an appropriate amount of sulfite/bisulfite solution, and possibly another alkaline solution, the target pH may be reached.

According to at least one example embodiment, the method comprises the step of increasing the pH of the pretreated biomass slurry to between 3 and 7, using the sulfite/bisulfite solution.

Hereby, the process can be adapted based on the pH with regards to preferred environment for various enzymes. For example, a relatively high pH may be desired if cellulolytic bacteria or fungi is used in downstream process steps.

It should be noted that SO2 can be retrieved from other process steps besides the pretreatment arrangement, e.g. the hydrolysis step or any fermentation step, or any auxiliary equipment arranged in between, e.g. a flash tank. The SO2 retrieved from the process is typically excess gaseous SO2.

According to at least one example embodiment, the step of treating the SO2 gas stream with an alkaline solution is performed in an SO2 scrubber.

Hereby, a well-suited equipment can be used to produce the sulfite/bisulfite solution. An SO2 scrubber is moreover relatively easy to implement in the process as it is adapted for separated streams of SO2 and alkaline solution. Moreover, the resulting sulfite/bisulfite solution is relatively easy withdrawn from the SO2 scrubber and can thus be used in the process accordingly.

According to at least one example embodiment, the method comprises the step of separation of the pretreated biomass slurry to remove a liquid fraction.

For example, the liquid fraction may be removed from the process, and the solid fraction may be used in the downstream process steps. Hereby, the process can be carried out in a more efficient manner. It should be noted that in embodiments with such separation, the supply of the sulfite/bisulfite solution according to option c) may be performed by supplying the sulfite/bisulfite solution to a process location between the reactor vessel and the separation.

According to at least one example embodiment, the method comprises a steam explosion step, preferably arranged downstream of the reactor vessel.

Hereby the biomass material is further disintegrated facilitating the degradation process.

According to at least one example embodiment, the method comprises subjecting the fermentable sugars of the hydrolysate to fermentation in an aqueous liquid utilizing at least one fermenting microorganism under conditions in which at least a part of the fermentable sugars is fermented into a primary target chemical.

Such fermentation may e.g. be carried out in a fermentation unit or fermentation vessel. The primary target chemical may e.g. be ethanol.

Moreover, any liquid withdrawn from any moisture adjusting step in the process may be recirculated into the process again, potentially treated in a sulfite/bisulfite treatment unit to form a separated gas stream including SO2, and/or be provided back into the system in the same way as the sulfite/bisulfite solution (any of options a)-d)).

### BRIEF DESCRIPTION OF THE DRAWINGS

The various aspects of the present invention, including its particular features and advantages, will be readily understood from the following detailed description and the accompanying drawings, in which:
Fig. 1 illustrates a pretreatment arrangement according to an exemplary embodiment of the present invention;
Fig. 2 schematically illustrates the steps of a method for controlled pretreatment to an exemplary embodiment of the present invention.
Fig. 3 schematically illustrates a system for treatment of lignocellulosic biomass according to the present disclosure, and
Fig. 4 is a graph showing the results of three measurement trials in accordance with an exemplary embodiment of the present invention.

### DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

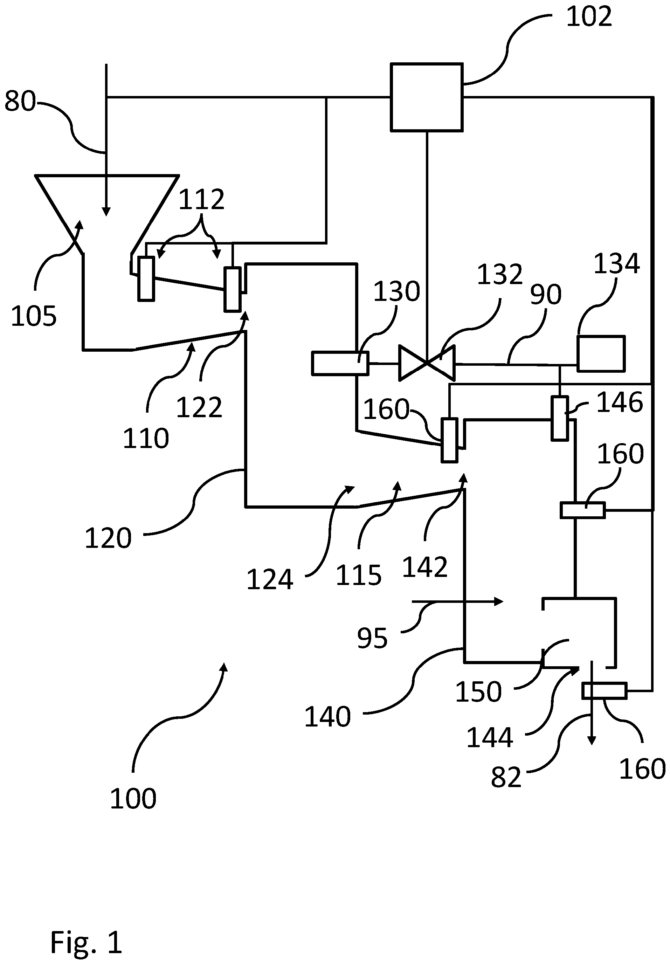

Fig. 1 illustrates a pretreatment arrangement 100 for pretreatment of lignocellulosic biomass comprising an inlet hopper 105 configured to receive lignocellulosic biomass material 80, an impregnation vessel 120 arranged downstream of the inlet hopper 105, and a reactor vessel 140 arranged downstream of the impregnation vessel 120. The impregnation vessel 120 comprises an upstream inlet 122 for receiving the lignocellulosic biomass material 80 from the inlet hopper 105 as a lignocellulosic biomass feed, and a downstream outlet 124 for discharging SO2-impregnated biomass to the reactor vessel 140. Correspondingly, the reactor vessel 140 comprises an upstream inlet 142 for receiving SO2-impregnated biomass from the outlet 124 of the impregnation vessel 120, and a downstream outlet 144 for discharging the biomass as a pretreated biomass slurry 82. In an outlet portion of the reactor vessel 140, a steam explosion configuration 150 is arranged.

The pretreatment arrangement 100 further comprises an SO2 injection arrangement 130 configured to supply an SO2 feed 90 to the lignocellulosic biomass in the impregnation vessel 120. The SO2 injection arrangement 130 includes a valve 132 having an adjustable opening configuration for varying the SO2 feed 90. Typically, the SO2 injection arrangement 130 is configured to supply the SO2 to the impregnation vessel 120 in gaseous form, but a configuration in which the SO2 is fed to the impregnation vessel 120 as a liquid is within the scope of the invention.

Thus, in more detail, the lignocellulosic biomass material 80 enters the pretreatment arrangement 100 via the inlet hopper 105, and is fed into the impregnation vessel 120 by means of a first plug screw feeder 110. In the impregnation vessel 120, the lignocellulosic biomass is treated or impregnated with SO2 by the use of the SO2 injection arrangement 130. Subsequently, the SO2 impregnated biomass from the impregnation vessel 120 is fed to the reactor vessel 140 by means of a second plug screw feeder 115. The first and second plug screw feeders 110, 115 secures an even flow of biomass material into the impregnation vessel 120 and reactor vessel 140, respectively. Moreover, the first and second plug screw feeders 110, 115 act as moisture adjusting units, here by compression which removes moisture from the biomass (dewatering). The pretreatment arrangement 100 is not limited to a specific type of inlet or feeding means, but any inlet or means for feeding biomass, known to those skilled in the art, may be used.

The lignocellulosic biomass may be, but is not limited to, hardwoods, softwoods, sugarcane bagasse, energy cane, corn stover, corn cobs, corn fibers, straw from rice, wheat, rye and other crop or forestry residues. As illustrated by the arrow 95, steam and/or additional catalysts may in embodiments be added to the reactor vessel 140 for certain pretreatment conditions.

In the reactor vessel 140, and during operation, the SO2 impregnated biomass from the impregnation vessel 120 is subject to an elevated temperature and pressure forming a biomass slurry. For example, the temperature in the reactor vessel 140 during pretreatment may be in the interval of 185 to 225°C, such as 200 to 215 °C. For example, the pressure in the reactor vessel 140 during pretreatment may be in the interval of 10 to 25 bar, such as 15 to 20 bar. Typically, the reactor vessel 140 has a circular or oval cross-section. The reactor vessel 140 may e.g. be cylindrical. In preferred embodiments, the reactor vessel 140 has a rotational symmetry with respect to a longitudinal center line.

The biomass content in the reactor vessel 140 can be divided into a gas phase arranged above the biomass slurry level, and a biomass slurry phase below the biomass slurry level in which the reactions for degrading or partially degrading the lignocellulosic biomass take place. During such reactions, and particularly when additional steam 95 or gases are introduced into the reactor vessel 140, an excess amount of gases are concentrated in the gas phase of the reactor vessel 140. During degradation of the lignocellulosic biomass, chemical bonds are broken and may result in the liberation of volatile compounds (VOC) and gases. Such gases are typically inert gases; i.e. gases that have no or extremely low chemical reactivity with other substances. Examples of inert gases include e.g. nitrogen (N2) and carbon dioxide (CO2). In the embodiment shown in Fig. 1, where SO2 is used to catalyze the pretreatment reaction of the lignocellulosic biomass, SO2 may also become concentrated in the gas phase of the reactor vessel 140. For this reason, the reactor vessel 140 may further comprise a gas valve 146 configured to remove gas from the reactor vessel 140. The gas valve 146 may be operated by a flow control means (not shown) configured to adjust the outflow of gas from the gas valve 146. A controlled removal of gases may contribute to a more stable pretreatment process. Furthermore, any withdrawn SO2 from the reactor vessel 140, may be re-used as SO2 feed 90 to the impregnation vessel 120, preferably subsequent to treating the withdrawn gas from the reactor vessel 140 to any needed SO2 separation/cleaning operation.

The reactor vessel 140 may according to one example embodiment be a vertical reactor vessel. However, horizontal, as well as inclined, reactor vessels are also conceivable for the purpose of the present disclosure. As illustrated in Fig. 1, the reactor vessel 140 is a vertical reactor vessel comprising an upper portion comprising the inlet 142, and a lower portion comprising the outlet 144. The biomass thus flows from the inlet 142 to the outlet 144 by means of gravity, and no additional means to increase the flow of biomass within the reactor vessel 140 is required. The gases formed in the reactor vessel 140 during the pretreatment reactions, and any superfluous SO2 assemble in the upper portion of the reactor vessel 140, i.e. above the biomass slurry level. The gas valve 146 is therefore arranged in the upper portion of the reactor vessel 140 to secure efficient removal of gas (not slurry) from the reactor vessel 140.

As previously described, an unstable pretreatment is less efficient, i.e. provides a lower yield, and also leads to problems such as build-up of deposits on the inner walls of the reactor vessel. The present inventors have realized that problems with unstable pretreatment conditions can be reduced if the pretreatment arrangement 100 is operated based on a desired pH downstream of the impregnation vessel 120, and more specifically, in order to reach the desired pH, adjusting the SO2 feed based on at least the amount, and dry content, of lignocellulosic biomass input to the pretreatment arrangement 100.

In Fig. 1, various alternatives of measuring the pH along the pretreatment process are provided. In more detail, the pretreatment arrangement 100 comprises at least one pH measuring unit 160 arranged and configured to measure the pH downstream of the impregnation vessel 120. In Fig. 1, three positions to measure the pH are exemplified, of which one or more may be utilized when practicing the invention. In a first pH measuring position, pH is measured on the SO2 impregnated biomass prior to feeding the same to the reactor vessel 140. For example, the pH measuring unit 160 may be arranged prior or at the inlet 142 to the reactor vessel 140, but after the outlet 124 of the impregnation vessel 120, e.g. prior, at or after the second plug screw feeder 115. In a second pH measuring position, pH is measured on the biomass slurry in the reactor vessel 140. Thus, the pH measuring unit 160 may be arranged and configured to measure pH in the reactor vessel 140. In a third pH measuring position, pH is measured on the pretreated biomass slurry 82 being discharged from the pretreatment arrangement 100. Here, the pH measuring unit 160 is arranged downstream of the reactor vessel 140. As an alternative, the pH may be measured on liquid withdrawn from the biomass during the pretreatment process after the impregnation vessel 120. It should be noted that other means and positions for measuring or determining the pH of the biomass downstream of the impregnation vessel 120 are possible without departing from the scope of the invention.

The pretreatment arrangement 100 may further comprise measuring means/units for measuring e.g. the temperature and pressure in the inside of the impregnation vessel 120 and the reactor vessel 140, respectively.

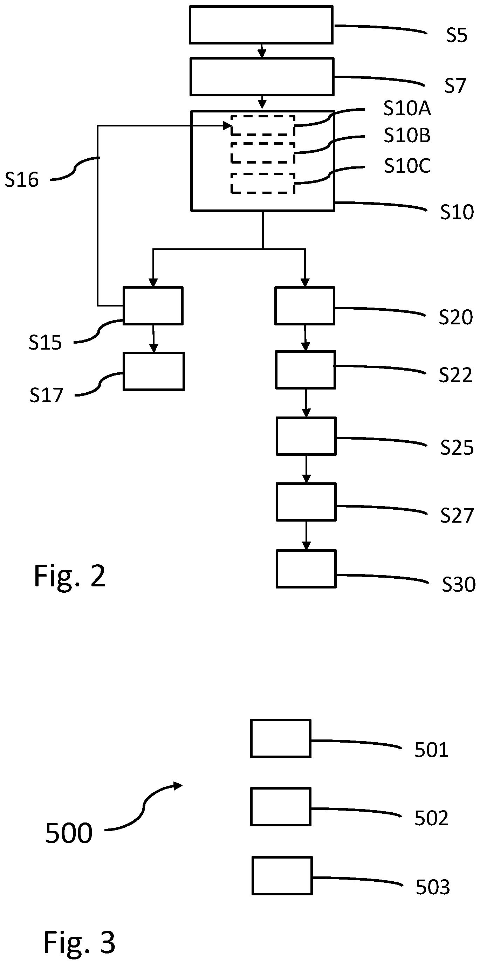

The invention will now be described with reference to the flow chart presented in Fig. 2. Fig. 2 schematically illustrates the steps of a method for controlled pretreatment of lignocellulosic biomass according to at least one example embodiment of the invention. The pretreatment arrangement, and any of its equipment, components and functions, may e.g. be those described with reference to Fig. 1.

First, the main steps of the method will be described, followed by a more detailed description including further optional steps. In step S10, a lignocellulosic biomass material is pretreated in a pretreatment arrangement, the pretreating comprising at least the sub-step S10A of impregnating the lignocellulosic biomass with an SO2 feed in an impregnation vessel of the pretreatment arrangement. Sub-step S10A may comprise supplying the SO2 to the impregnation vessel in gaseous form, 10 and the step S10 may comprises operating the impregnation vessel at a temperature of between 20 °C and 60 °C. In step S20, a number of process parameters of the pretreatment, are collected, the process parameters include at least a feed parameter related to the total amount of lignocellulosic biomass input to the pretreatment arrangement (e.g. lignocellulosic biomass 80 of Fig. 1), and a dry matter parameter related to the dry matter content of lignocellulosic biomass input to the pretreatment arrangement. In step S30, the SO2 feed is adjusted in response to the process parameters. In other words, the SO2 feed is adjusted in response to at least the feed parameter and the dry matter parameter. In embodiments, the method comprises setting a lower limit for the SO2 feed, so as to enable a continuous flow of gas into the impregnation vessel during the pretreatment.

The feed parameter may e.g. be measured by measuring the weight of lignocellulosic biomass input to the pretreatment arrangement. Alternatively, the feed parameter is estimated based on known volume of lignocellulosic biomass input to the pretreatment arrangement. As another alternative, the amount of lignocellulosic biomass input to the pretreatment arrangement is known on beforehand, e.g. by being specified by a biomass provider. The feed parameter may be based on an average of a plurality of measurements/estimations.

Correspondingly, the dry matter parameter may e.g. be measured by measuring the dry content directly via a measurement means, e.g. an IR-based measuring device, or estimated based on known characteristics of the lignocellulosic biomass input to the pretreatment arrangement. It should be noted that the pretreatment arrangement may comprise process steps which change the dry matter content of the lignocellulosic biomass prior to feeding the same to the impregnation vessel, and in such cases, the dry matter content may instead be measured/estimated subsequent to such process step, but still upstream of the impregnation vessel. For example, the method may comprise the step S5 of adjusting the moisture in the lignocellulosic biomass material prior to impregnating the lignocellulosic biomass with an SO2 feed in the impregnation vessel. The adjusting of moisture may e.g. be embodied by dewatering the lignocellulosic biomass material e.g. in a compressing process. The method may further comprise the step S7 of measuring the dry matter content of the lignocellulosic biomass prior to, and/or subsequent to, the step S5, and wherein at least one of the measured dry matter contents is used as dry matter parameter. Use of such measurement/estimation for the dry matter parameter is still considered to be within the definition of it being related to the dry matter content of lignocellulosic biomass input to the pretreatment arrangement. Alternatively, the dry matter parameter may be defined as being related to the dry matter content of lignocellulosic biomass input to the impregnation vessel. The dry matter parameter may be based on an average of a plurality of measurements/estimations.

The method is preferably automated and adapted for continuous operation. In embodiments, step S20 of collecting comprises monitoring the process parameters in a continuous or semi-continuous manner and the step of adjusting S30 comprises automatically adjusting the SO2 feed in response to the monitored process parameters.

The step S30 may preferably be adapted to meet a desired pH downstream of the impregnation vessel, e.g. the pH of the lignocellulosic biomass discharged from the pretreatment arrangement. Thus, the step S30 of adjusting the feed of SO2 in response to the measure process parameters includes adjusting the feed of SO2 based on the measured pH, to reach a desired pH. In practice, the step S30 is typically carried out to increase or decrease the amount of SO2 fed to the impregnation vessel, i.e. by adjusting an opening of a gas valve arranged and configured to regulate the flow of SO2. The flow of SO2 may be measured by suitable means, such as a flow meter.

In a more detailed description of Fig. 2, the step S10 comprises the sub-step S10B of subjecting the SO2 impregnated biomass from the impregnating step S10A to an elevated temperature and pressure in a reactor vessel of the pretreatment arrangement to form a pretreated biomass slurry. Optionally, in step S10C, the biomass slurry is subject to a steam explosion, preferably arranged downstream, or in an end section of, the pretreatment arrangement or reactor vessel.

In step S25, pH of the SO2 impregnated biomass from the impregnating step and/or the pretreated biomass slurry is measured. There are several ways of determining pH of the biomass downstream of the impregnation vessel as previously described. In a subsequent step S27, it is determined whether or not the measured pH is within a predetermined range of the desired pH. Based on the outcome of step S27, step S30 is adapted. For example, if it is determined that the measured pH is outside of the predetermined range of the desired pH, the SO2 feed is adjusted in response to such determination, with the aim to steer the pH towards the desired pH. As mentioned for the feed parameter and the dry matter parameter, the pH may be continuously measured, and/or be based on an average of a plurality of measurements, and the step of adjusting S30 may comprise automatically adjusting the SO2 feed in response to the measured pH and/or in response to the deviation of the measured pH to the desired pH. The desired pH may e.g. be in the range of 1 to 4, preferably 1.2 to 2, and more preferably 1.3 to 1.8.

The method may further comprise the step S22 of determining a relation of the feed parameter and the dry matter parameter, and adapting the step S30 such that the SO2 feed is adapted in response to the determined relation. The inventors have realized that the SO2 feed (e.g. in kg SO2 / h) is preferably related to the dry matter of the biomass in order to reach the desired pH. Thus, by using the above mentioned relation of the feed parameter and the dry matter parameter, a more straightforward relation to the SO2 feed can be achieved. The method may e.g. comprise (e.g. included in step S30), the sub-step of determining whether or not the determined relation is within a predetermined reference relation for the pretreatment, and adjusting the SO2 feed in response to determining that the determined relation is outside of the predetermined relation. For example, such adjustment may be performed if the determined relation is outside of the predetermined relation by more than 5%, e.g. by more than 10% or more than 15 % or more than 20 %. The predetermined reference relation may be a predetermined set value or interval, or, alternatively, be predetermined to be for example the relation of the feed parameter and the dry matter parameter at an initial stage of the pretreatment process.

According to at least one example embodiment, the method comprises step S15 of retrieving at least a portion of SO2 from the pretreatment arrangement, and the step S16 of re-using the retrieved SO2 in the controlled pretreatment of the lignocellulosic biomass, which e.g. is embodied by the gas valve of 146 in Fig. 1. Additionality or alternatively, the retrieved SO2 can be used in a downstream process of the pretreatment arrangement, as symbolised by step S17.

It should be noted that step S30 may be based on further process parameters than those mentioned here. For example, the process parameters may include an SO2 parameter indicating the amount of SO2, or concentration of SO2 gas, in the impregnation vessel. Thus, the feed of SO2 in step S30 may be adjusted based on the SO2 parameter. Thus, the method may comprise the step of determining (e.g. by measuring) the amount of SO2, or concentration of SO2 gas, in the impregnation vessel. This may be of particular value when more than one source of SO2 are used for impregnating the lignocellulosic biomass, e.g. by liquid impregnation prior to or after the impregnation vessel.

Turning back to Fig. 1, the pretreatment arrangement 100 further comprises a control unit 102 configured to perform one or more of the method steps described with reference to Fig 2.

Thus, the control unit 102 is configured to at least collect a number of process parameters of the pretreatment arrangement 100, which process parameters include at least the previous mentioned feed parameter and dry matter parameter. The feed parameter may e.g. be determined by measuring the weight or volume of the lignocellulosic biomass material 80 which enters the pretreatment arrangement 100 via the inlet hopper 105. The dry matter parameter may e.g. be determined by measuring means 112 arranged and configured to determine the dry matter content of the lignocellulosic biomass prior to, and/or subsequent to, the first plug screw feeder 110.

Furthermore, the control unit 102 is configured to adjust the valve 132 of the SO2 injection arrangement 130, and thereby adjust the SO2 feed, in response to the process parameters. As also shown in Fig. 1, the control unit 102 is connected to the pH measuring unit(s) 160, and thus configured to adjust the SO2 feed in response to the process parameters, and to meet a desired pH downstream of the impregnation vessel 120.

The valve 132 may thus together with the control unit 102 be configured to adjust the SO2 feed through the injection arrangement 130 in response to the feed parameter and dry matter parameter, and, possibly other process parameters, to meet a desired pH downstream of the impregnation vessel 120. Stated differently, the pretreatment arrangement 100 may comprise a SO2 feed control means, wherein the SO2 feed control means comprises at least the valve 132 and control unit 102, and possibly other conventional control means arranged to communicate with and collect signals from any the measuring or acquiring means (i.e. to collect at least the feed parameter and dry matter parameter) and to send control signals to the valve 132 or, alternatively, to an intermediate valve adjustment device (not shown), in order to adjust the opening of the valve 132. Such SO2 feed control means may preferably comprise automated control means, such as computer control means, suitable for online or inline pretreatment control.

The valve 132 is arranged in fluid communication with an SO2 supply 134, so as to secure proper accessibility of SO2. The valve 132 typically has an adjustable opening configuration, i.e. the degree of opening of the valve 132 and/or the time of opening of the valve 132 is adjustable. The valve 132 may be operable between an open and closed configuration in a "1/0", stepwise, or stepless manner. In some embodiments, the valve 132 is configured to be operable between an open and a closed position. A flexible, yet controlled system is thereby achieved which allows for quick adjustments or "corrections" of impregnation conditions within the impregnation vessel 120. In alternative embodiments, the valve 132 is configured to supply and SO2 feed continuously to the impregnation vessel 120.

The injection arrangement 130 and/or valve 132 may be attached to the impregnation vessel 120 or may be connected to the impregnation vessel 120 by means of a pipe, a tube, or another connecting means. The injection arrangement 130 may also be arranged in the impregnation vessel 120 or extend into the impregnation vessel 120. In Fig. 1, the valve 132 is arranged outside of the impregnation vessel 120 and is connected to the injection arrangement 130 inside the impregnation vessel 120 by means of e.g. a pipe.

The present disclosure is not limited to a specific type of valve or injection arrangement, and, as evident to the skilled person, the type of valve and injector(s) used may depend for example on the process conditions and method for pretreatment and whether the impregnation vessel is used for large-scale or small-scale pretreatment processes. In particular, the size and shape of the reactor vessel may affect which type of valve and injection arrangement that are suitable. Exemplary valves include solenoid valve, needle valve, pinch valve, ball valve, globe valve, knife valve, spool valve, butterfly valve, choke valve, diaphragm valve, membrane valve, gate valve, piston valve, and plug valve. In preferred embodiments, the valve 132 is a control valve, i.e. a valve with variable and controllable degree of opening. The control valve is configured with an adjustable, stepwise or stepless, opening for SO2 feed, not just a 1/0 or on/off ("1/0") mode. In embodiments, the control valve is a globe valve, a ball valve, or a needle valve.

In embodiments, the pretreatment arrangement 100 further comprises an optional flow meter (not shown) configured to measure the flow of SO2 through of injection arrangement 130. In such cases, the SO2 feed is also varied in response to the measured flow of SO2. For example, if the flow meter indicates that there is an improper flow of SO2 (either too much or too low), the valve 132 may be opened or closed in response to such indications. This may be beneficial to secure that energy is not lost or "wasted" during the impregnation. A cost-efficient and precisely controlled approach is thereby achieved. The valve 132 and the flow meter may be arranged within the same unit or may be connected by means of a pipe or tube. The flow meter may alternatively be arranged upstream or downstream of the valve 132. The pretreatment arrangement 100 is not limited to the use of a specific flow meter, but any type of flow meter suitable for measuring the flow of SO2 feed to the impregnation vessel 120 may be used. The flow meter may e.g. be selected from a differential pressure flow meter, a variable flow meter, a Coriolis flow meter, an ultrasonic flow meter, an optical flow meter or a thermal dispersion flow meter. In embodiments, the flow meter is a rotameter. The rotameter is a type of variable flow meter that measures the volumetric flow rate of a gas flow. A rotameter typically comprises a tapered tube with a "float"; i.e. at weight that is pushed upwards when the gas flow increases. The flow rate is measured by allowing the cross-sectional area that the gas travels through to vary.

As mentioned previously, the SO2 feed control is configured to control the SO2 feed to the impregnation vessel 120 such that a desired pH is met downstream of the impregnation vessel 120. This is e.g. beneficial to secure stable reaction conditions throughout the pretreatment arrangement 100, efficient pretreatment performance and energy-efficient pretreatment. A cost-efficient and adequately controlled pretreatment process can thereby be achieved.

The pretreatment arrangement 100 of the present disclosure may be used for various types of pretreatment methods including both hydrothermal, chemical, physical and biological methods.

With reference to Fig. 3, the present disclosure further provides a system 500 for treatment of lignocellulosic biomass comprising a pretreatment arrangement 501 for pretreatment of lignocellulosic biomass according to the embodiments of Fig. 1 and Fig. 2, a hydrolysis unit 502 arranged downstream of and in fluid communication with the pretreatment arrangement 501, and optionally, a fermentation unit 503, such as a fermentation vessel, arranged downstream of and in fluid communication with the hydrolysis unit 502. The system 500 may comprise additional units and components known to those skilled in the art. For example, a separation unit may be arranged between the pretreatment arrangement 501 and the hydrolysis unit 502, and/or between the hydrolysis unit 502 and the fermentation unit 503.

In the hydrolysis unit 502, the pretreated biomass is subject to enzymatic hydrolysis by means of saccharification enzymes. Fermentation of the hydrolysate into a target chemical is typically performed by means of fermenting organism, such as bacteria and/or yeast in the fermentation unit 503. The system 500 may also comprise a product recovery unit, such as distillation or ion exchange chromatography, arranged downstream of and in fluid communication with the fermentation unit 503.

### EXAMPLES

Pretreatment of biomass, spruce sawdust, was performed by Sekab E-Technology in the Biorefinary Demo Plant (BDP), Ornskoldsvik, Sweden. The biomass was pretreated in continuous mode in a pre-treatment arrangement having a 200 1 impregnation vessel and a 20 1 reactor vessel configured for steam explosion. Three measurement trials, trial 1-3, were carried out.

For all three trials, sulfur dioxide, SO2, (g) was used to impregnate the biomass in the impregnation vessel at 30-40 °C for 15-20 min. The subsequent pretreatment in the reactor vessel was carried out for 12-14 min at a pressure indicated in table 1. After the pretreatment of the biomass, the pH of the pretreated biomass slurry was measured by removing a liquid fraction of the pretreated biomass slurry by filtration.

The amount of added SO2, the SO2-feed, was varied and for each setting, at least two pH measurements were made. The pH was determined by laboratory analysis. Moreover, the dry matter content of the biomass input was determined for each trial by laboratory analysis, and it varied according to table 1 (from about 50 percent (w/w) to about 60 percent (w/w)). The total amount of biomass input to the pretreatment arrangement varied between 60 kg/h to 70 kg/h as specified in table 1.

**Table I**

| | Biomass input (kg/h) | Pressure reactor vessel (barg) | Dry matter content (% w/w) |
|---|---|---|---|
| Trial 1 | 60 | 15 | ∼57 |
| Trial 2 | 60 | 15 | ∼50 |
| Trial 3 | 70 | 14 | ∼51 |

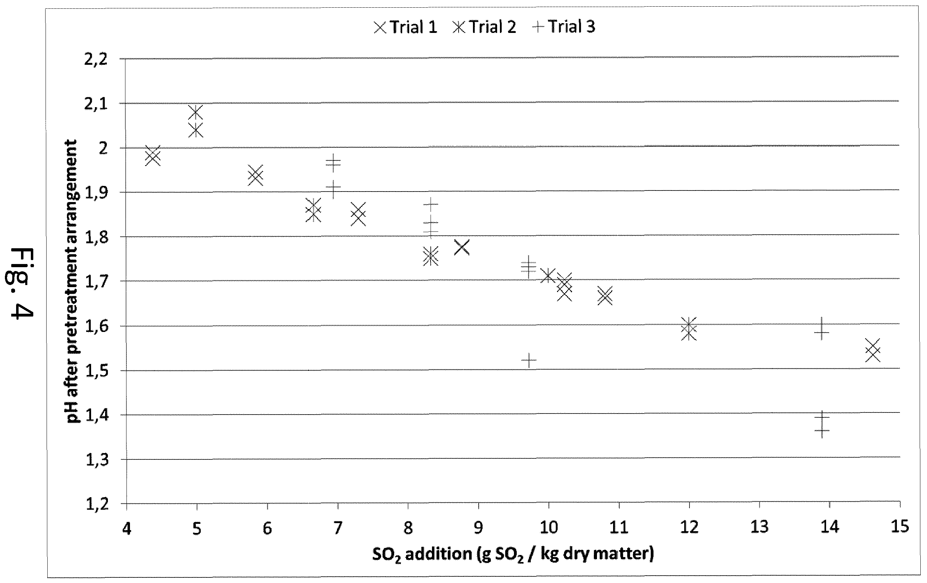

In Fig. 4, the pH of the biomass slurry after the reactor vessel (i.e. after the pretreatment) were plotted against the added SO2 (the SO2-feed, gSO2 / kg dry matter biomass), corrected with respect to biomass input and the moisture content of the untreated biomass. As can be seen from Fig. 4, there is a clear relation between the SO2 feed and the pH of the biomass. From this relation, the inventors realized that a linear relation between the SO2 feed and the pH of the biomass, based on known data of biomass input to the pretreatment arrangement and the dry matter content of the biomass, is a sufficient approximation to be used when regulating the SO2 feed to reach a desired pH after the reactor vessel of the pretreatment arrangement.

According to at least one example embodiment of the invention, the step of adjusting the SO2 feed comprises adjusting the SO2 feed in response to a known relation between the feed parameter, the dry matter parameter, and the resulting pH after the pretreatment. Such relation may e.g. be the linear relation between the SO2 feed and the pH of the biomass after the pretreatment as shown in Fig. 4.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for controlled pretreatment of lignocellulosic biomass comprising the steps of:
Pretreating (S10) a lignocellulosic biomass material in a pretreatment arrangement, the pretreating comprising impregnating (S10A) the lignocellulosic biomass with an SO2 feed in an impregnation vessel of the pretreatment arrangement;
collecting (S20) a number of process parameters of the pretreatment, which process parameters include at least a feed parameter related to the total amount of lignocellulosic biomass input to the pretreatment arrangement, and a dry matter parameter related to the dry matter content of lignocellulosic biomass input to the pretreatment arrangement; and
adjusting (S30) the SO2 feed in response to the process parameters.

2. The method according to claim 1, wherein the step of adjusting (S30) the SO2 feed in response to the process parameters is adapted to meet a desired pH downstream of the impregnation vessel.

3. The method according to any one of claims 1-2, wherein the step of pretreating (S10) a lignocellulosic biomass material in a pretreatment arrangement comprises subjecting (S10B) SO2 impregnated biomass from the impregnating step to an elevated temperature and pressure in a reactor vessel of the pretreatment arrangement to form a pretreated biomass slurry.

4. The method according to claims 2 and 3, further comprising the steps of measuring (S25) pH of SO2 impregnated biomass from the impregnating step and/or the pretreated biomass slurry, determining (S27) whether or not the measured pH is within a predetermined range of the desired pH, and adjusting (S30) the SO2 feed if the measured pH is outside of the predetermined range of the desired pH.

5. The method according to any one of claims 2 and 4, wherein the desired pH is in the range of 1 to 4, preferably 1.2 to 2, and more preferably 1.3 to 1.8.

6. The method according to any one of the preceding claims, wherein the step of impregnating (S10A) the lignocellulosic biomass with an SO2 feed comprises supplying the SO2 to the impregnation vessel in gaseous form.

7. The method according to any one of the preceding claims, further comprising the step of determining (S22) a relation between the feed parameter and the dry matter parameter, and adjusting (S30) the SO2 feed in response to the determined relation.

8. The method according claim 7, wherein the step of adjusting (S30) the SO2 feed comprises determining whether or not the determined relation is within a predetermined reference relation for the pretreatment; and adjusting the SO2 feed in response to determining that the determined relation is outside of the predetermined reference relation, such as by more than 5%, e.g. by more than 10% or more than 15 %.

9. The method according to any one of the preceding claims, further comprising the steps of adjusting the moisture (S5) in the lignocellulosic biomass material prior to impregnating the lignocellulosic biomass with an SO2 feed in the impregnation vessel, and measuring (S7) the dry matter content of the lignocellulosic biomass prior to, and/or subsequent to, the adjusting moisture step, and wherein at least one of the measured dry matter contents is used as dry matter parameter.

10. The method according to any one of the preceding claims, wherein the step of pretreating (S10) comprises operating the impregnation vessel at a temperature of between 20 °C and 60 °C.

11. The method according to any one of the preceding claims, further comprising the steps of retrieving (S15) at least a portion of SO2 from the pretreatment arrangement, and re-using (S16) the SO2 in the controlled pretreatment of the lignocellulosic biomass, or using (S17) the SO2 in a downstream process of the pretreatment arrangement.

12. The method according to any one of the preceding claims, further comprising a steam explosion step (S10C), preferably arranged downstream, or in an end section of, the pretreatment arrangement.

13. The method according to any one of the preceding claims, wherein the process parameters further include an SO2 parameter indicating the amount of SO2, or concentration of SO2 gas, in the impregnation vessel.

14. A pretreatment arrangement (100) for pretreatment of lignocellulosic biomass material (80) comprising:
an impregnation vessel (120) having an upstream inlet (122) for receiving a lignocellulosic biomass feed, a downstream outlet (124) for discharging SO2-impregnated biomass, and an SO2 injection arrangement (130) configured to supply an SO2 feed to the lignocellulosic biomass in the impregnation vessel, the SO2 injection arrangement comprising a valve (132) having an adjustable opening configuration for varying the SO2 feed;
a reactor vessel (140) having an upstream inlet (142) for receiving SO2-impregnated biomass from the impregnation vessel, and a downstream outlet (144) for discharging the biomass as a pretreated biomass slurry;
a control unit (102) configured to collect a number of process parameters of the pretreatment arrangement, which process parameters include at least a feed parameter related to the amount of lignocellulosic biomass input to the pretreatment arrangement, and a dry matter parameter related to the dry matter content of lignocellulosic biomass input to the pretreatment arrangement,
wherein the control unit is further configured to adjust the valve of the SO2 injection arrangement, and thereby adjust the SO2 feed, in response to the process parameters.

15. The pretreatment arrangement according to claim 14, further comprising a pH measuring unit (160) arranged and configured to measure the pH downstream of the impregnation vessel, and wherein the control unit is configured to adjust the SO2 feed in response to the process parameters, and to meet a desired pH downstream of the impregnation vessel.

16. The pretreatment arrangement according to any one of claims 14-15, further comprising a moisture adjusting unit (110), e.g. a plug screw feeder (110), configured to adjust the moisture content of the lignocellulosic biomass material prior to the inlet of the impregnation vessel, and comprising a measuring means (112) arranged and configured to determine the dry matter content of the lignocellulosic biomass prior to, and/or subsequent to, the moisture adjusting unit, and wherein at least one of the measured dry matter contents is used as dry matter parameter.

17. The pretreatment arrangement according to any one of claims 14-16, further comprising a steam explosion configuration (150) arranged downstream, or in an outlet region of, the reactor vessel.

18. The pretreatment arrangement according to any one of claims 14-17, wherein the control unit is configured for performing the method steps of any one of claims 1-13.
